(19) Europäisches Patentamt
European Patent Office
Office européen des brevets

(11) Publication number : 0 306 094 B1

(12) # EUROPEAN PATENT SPECIFICATION

(45) Date of publication of patent specification : **29.01.92 Bulletin 92/05**

(51) Int. Cl.$^5$ : **C07C 69/716,** C07C 67/38

(21) Application number : **88201840.1**

(22) Date of filing : **29.08.88**

(54) **Process for the hydroformylation of certain acrylic acid derivatives.**

(30) Priority : **01.09.87 GB 8720510**

(43) Date of publication of application : **08.03.89 Bulletin 89/10**

(45) Publication of the grant of the patent : **29.01.92 Bulletin 92/05**

(84) Designated Contracting States : **BE CH DE FR GB LI NL**

(56) References cited :
**EP-A- 0 125 567**
**FR-A- 2 365 549**

(73) Proprietor : **SHELL INTERNATIONALE RESEARCH MAATSCHAPPIJ B.V.**
**Carel van Bylandtlaan 30**
**NL-2596 HR Den Haag (NL)**

(72) Inventor : **Drent, Eit**
**Badhuisweg 3**
**NL-1031 CM Amsterdam (NL)**
Inventor : **Breed, Antonius Johannes Maria**
**Badhuisweg 3**
**NL-1031 CM Amsterdam (NL)**

(74) Representative : **Tuijn, Jan Warnaar et al**
**Shell Internationale Research Maatschappij B.V., Patents, Licensing & Trade Marks Division, P.O. Box 302**
**NL-2501 CH The Hague (NL)**

EP 0 306 094 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the hydroformylation of certain acrylic acid derivatives.

British patent specification number 1,586,805 discloses a process for the preparation of $C_{1-18}$ alkyl α-formylpropionates by the hydroformylation of $C_{1-18}$ alkyl acrylates at a pressure of from 100 to 700 bars and a temperature of from 50 to 200°C, in the presence of a rhodium complex which contains, as a ligand bound to rhodium, a phosphorus compound of the general formula

$$P[(O)_nR]_3$$

where the radicals R are identical or different alkyl, aromatic, aralkyl or cycloalkyl radicals and n is 1 or zero. Triphenylphosphine and triphenylphosphite are specifically disclosed on page 2, lines 31 to 34 as suitable phosphorus compounds. However, the two Examples only illustrate the use of triphenylphosphine. Both Examples illustrate the hydroformylation of methyl acrylate at a pressure of 280 bars and a temperature of 105°C or above.

In a review paper entitled "Advances in the Hydroformylation of Olefins Containing Functional Groups", published in the Journal of Molecular Catalysis, 40 (1987) 129-182, it is stated on page 164 that alkyl acrylates have been frequently hydroformylated by rhodium catalysts at high pressure. However, it is also stated that low pressure hydroformylation of ethyl acrylate (≦ 0.4MPa) using $HRh(CO)(PPh_3)_3$ in the presence of an excess of $PPh_3$ at 80°C failed remarkably.

One skilled in the art would be inclined to conclude that alkyl acrylates can only be hydroformylated at high pressures when using the rhodium catalysts described in British patent specification number 1,586,805.

Surprisingly it has now been found that certain acrylic acid derivatives, including $C_{1-18}$ alkyl acrylates, may be hydroformylated at low pressure using a catalyst comprising a rhodium compound and a triphenylphosphite.

Accordingly, the present invention provides a process for the hydroformylation of a compound of general formula

$$R_1 - CH = \overset{H}{\overset{|}{C}} - \overset{O}{\overset{\|}{C}} - O - R_2 \qquad I$$

wherein $R_1$ represents a hydrogen atom or an alkyl group having from 1 to 6 carbon atoms and $R_2$ represents an alkyl group having from 1 to 18 carbon atoms, which comprises hydroformylating the compound of general formula I with carbon monoxide and hydrogen in the liquid phase in the presence of a homogeneous catalyst system comprising

a) a rhodium compound and

b) one or more triphenylphosphites, the phenyl groups of which may optionally be substituted by halogen, alkyl having from 1 to 6 carbon atoms, alkoxy having from 1 to 6 carbon atoms, carboxy or cyano, at a temperature in the range of from 50 to 100°C and a pressure in the range of from 3 to below 100 bars.

The process according to the invention has been shown to afford aldehydes at a very high conversion rate ( ≧ 5200 mol aldehyde/g at Rh.hr). Since high pressures are not required by the process according to the invention, it is thus particularly advantageous.

In view of the disclosure in British patent specification number 1,586,805 and Journal of Molecular catalysis, 40 (1987) 129-182, the process according to the invention is very surprising. Indeed when the conditions of the process of the invention were performed using triphenylphosphine or tri-(n)-butylphosphite instead of a triphenylphosphite, only very low conversion rates were observed.

The process according to the invention has been shown to proceed with very high selectivity for the iso-aldehyde. It is thus particularly advantageous for preparing alkyl α-formylpropionates which are useful in the preparation of fine chemicals and/or pharmaceuticals.

Preferably $R_1$ represents a hydrogen atom.

When $R_1$ represents a hydrogen atom, $R_2$ preferably represents an ethyl, n-propyl, isopropyl, isobutyl, n-butyl, or more preferably, a methyl group.

The rhodium compound used in the process according to the invention may be, for example, rhodium acetate, rhodium di(carbonyl)acetylacetonate, 1,5-cyclooctadiene-rhodium (I) acetate, 1,5-cyclooctadiene-rhodium (I) acetyl acetonate, dirhodium octacarbonyl, rhodium nitrate, rhodium chloride, tetrarhodium dodeca carbonyl or hexa rhodium hexadeca-carbonyl.

Preferably the number of gram atoms of rhodium used per mole of compound of general formula I is in the range of from 0.001-0.1.

Preferably the or each triphenylphosphite used in the process according to the invention is selected from

triphenylphosphite, di(phenyl)p-chlorophenylphosphite di(p-chlorophenyl)phenyl phosphite, tri(p-chlorophenyl) phosphite, di(phenyl)p-cyanophenyl phosphite, di(p-cyanophenyl) phenylphosphite, tri(p-cyanophenyl)phosphite, di(phenyl)p-methoxyphenylphosphite, di(p-methoxyphenyl)phenylphosphite, tri(p-methoxyphenyl)phosphite, di(phenyl)tolylphosphite, phenyl di(tolyl)phosphite, tri(tolyl)phosphite, di(phenyl)p-carboxyphenylphosphite, di(phenyl)o-chlorophenylphosphite, phenyl di(o-chlorophenyl)-phosphite and tri(o-chlorophenyl)phosphite.

More preferably, the or each triphenylphosphite used is selected from triphenylphosphite, diphenyl(p-chloro- phenyl)phosphite, diphenyl(p-methoxyphenyl)phosphite, diphenyl(p-cyanophenyl)phosphite and diphenyl(p-tolyl)phosphite. Triphenylphosphite itself is most preferred.

It will be appreciated that the hydrogen and carbon monoxide used in the process according to the invention may be undiluted or diluted with one or more inert gases, such as nitrogen, helium and carbon dioxide.

The molar ratio of hydrogen to carbon monoxide is preferably in the range of from 2 : 1 to 1 : 2, more preferably about 1 : 1.

Generally speaking the amount of the triphenylphosphite based on rhodium as central atom of the active complex, that must be present for attractive results is at least the stoichiometric amount, which corresponds to the preferred rhodium complexes $HRhCO[P(O\ Phenyl)_3]_3$ and $ClRhCO[P\text{-}(O\ Phenyl)_3]_2$.

However, an excess of the triphenylphosphite over the stoichiometric amount for complex formation i.e. over the amount actually complexed, has an advantageous effect on the yield of the desired isoaldehyde compound. This excess of the triphenylphosphite in free form, amounts advantageously to from 2-10, preferably from 2-6 and more preferably from 3-5 moles per mole of rhodium compound.

The reaction temperature is preferably in the range of from 55-75°C. The pressure is preferably in the range of from 15 to 60 bars.

The residence period can vary from about a couple of minutes to several hours, and as will be appreciated, this variable will be influenced to a certain extent by the reaction temperature, the choice of the specific reactant, the specific catalyst composition, the total synthesis gas pressure and the partial pressure exerted by its components.

A solvent is not generally essential in the process according to the invention, since usually one of the reactants or products can also serve as solvent. However, in certain circumstances the use of a solvent is desirable and practical. One can employ any normally-liquid organic solvent which does not interfere to any substantial degree with the desired hydroformylation reaction under the operative conditions employed. More preferably an apolar solvent is used, if any. Examples of such solvents include the saturated hydrocarbons, such as pentanes, naphtha, kerosene, cyclohexane etc. as well as the aromatic hydrocarbons, ethers, ketones, amides and nitriles. More specific examples are benzene, xylenes, toluene, diethyl ether, acetophenone, cyclohexanone, benzonitrile, N,N-dimethylacetamide, N,N-dimethylformamide and p-dioxan. More preferably toluene, benzene or xylenes will be used.

The preparation of the catalyst system to be used in the process of the present invention may be carried out by applying conventional techniques.

One suitable method is to combine the rhodium salt of an organic acid with the ligand, e.g. triphenylphosphite in the liquid phase. The valence state of rhodium may then be reduced by hydrogenating the solution prior to the use of the catalysts therein.

Alternatively, the catalysts may be prepared from a carbon monoxide complex of rhodium. E.g. one could start with dirhodium octacarbonyl and by heating this substance with the ligand, the ligand will replace one or more of the carbon monoxide molecules, thus producing the desired catalyst. It is also possible to start with the ligand of choice and rhodium metal or an oxide of rhodium and prepare the active catalyst species in situ during the hydroformylation reaction.

The process of the present invention may be carried out batchwise, semi continuously or continuously. When operating batchwise, the catalytic system, the starting acrylate compound and solvent are charged to a reactor to form a liquid phase therein, the reactor is pressurized with carbon monoxide and hydrogen and heated to the desired temperature. When operating continuously, the liquid components can be charged to the reactor continuously to form a liquid phase therein and the carbon monoxide/hydrogen continuously introduced into the reactor to contact the liquid phase containing the catalyst. The gaseous reactants can be withdrawn from the reactor as a separate effluent, cooled depressurized and the carbon monoxide and hydrogen may be recycled.

The following examples illustrate the invention.

All experiments were carried out in a 300 ml magnetically stirred Hastelloy C autoclave ("Hastelloy" is a trade mark). The reaction mixtures obtained were analyzed by means of gas-liquid chromatography.

Example 1

The autoclave was charged with rhodium di(carbonyl) acetyl acetonate (0.1 mmol), 20 ml methyl acrylate, 40 ml toluene and 0.3 mmol triphenylphosphite.

The autoclave was flushed with an equimolar mixture of hydrogen and carbon monoxide and then pressurized to 60 bar. The reaction mixture was heated up to 70°C, whilst maintaining the before-mentioned pressure.

After a total reaction time of 0.5 hr, the reaction mixture was cooled to room temperature and analyzed. The methyl acrylate conversion rate was calculated as being 5800 molCO/gat Rh.hr, while the selectivity for total aldehydes was 90% and for isoaldehyde was 93%.

Example 2

In rather the same way as described under example 1, an experiment was carried out, using 1 mmol triphenylphosphite instead of 0.3 mmol. A conversion rate of 5250 molCO/gat Rh.hr was calculated, while the selectivity for total aldehydes was 90% and for isoaldehyde was 96%.

Example 3

In rather the same way as described under example 1, an experiment was carried out, except that the total pressure was kept constant at 18 bar, while the temperature was 70°C.

After a total reaction time of 3.5 hr, a methyl acrylate conversion rate was calculated as being 1200 mol aldehyde/gat Rh.hr. The selectivity for total aldehydes was 95% and the selectivity for isoaldehyde was 88%.

Comparative Example A-C

In rather the same way as described under example 1, experiments were carried out, using 0.3 mmol triphenylphosphine, 0.3 mmol tri-(n)-butyl phosphite and 0.2 mmol diphenyl(pyridyl) phosphine respectively instead of 0.3 mmol of triphenylphosphite, showing after a reaction time of 5, 4 and 5 hours respectively, a calculated conversion rate of 100, 180 and 300 mol/gat Rh.hr, while selectivities for total aldehydes of 90%, > 30% and 80% were found and selectivities for isoaldehyde of 90%, 70% and 90% respectively were found.

**Claims**

1. A process for the hydroformylation of a compound of general formula

$$R_1 - CH = \overset{H}{\overset{|}{C}} - \overset{O}{\overset{||}{C}} - O - R_2 \qquad I$$

wherein $R_1$ represents a hydrogen atom or an alkyl group having from 1 to 6 carbon atoms and $R_2$ represents an alkyl group having from 1 to 18 carbon atoms, which comprises hydroformylating the compound of general formula I with carbon monoxide and hydrogen in the liquid phase in the presence of a homogeneous catalyst system comprising

a) a rhodium compound and

b) one or more triphenylphosphites, the phenyl groups of which may optionally be substituted by halogen, alkyl having from 1 to 6 carbon atoms, alkoxy having from 1 to 6 carbon atoms, carboxy or cyano, at a temperature in the range of from 50 to 100°C and a pressure in the range of from 3 to below 100 bars.

2. A process as claimed in claim 1, in which $R_1$ represents a hydrogen atom.

3. A process as claimed in claim 2, in which $R_2$ represents a methyl group.

4. A process as claimed in any one of claims 1 to 3, in which the number of gram atoms of rhodium used per mole of compound of general formula I is in the range of from 0.001 to 0.1.

5. A process as claimed in any one of claims 1 to 4, in which the or each triphenylphosphite used is selected from :

triphenylphosphite,
diphenyl(p-chlorophenyl)phosphite,
diphenyl(p-methoxyphenyl)phosphite,

diphenyl(p-cyanophenyl)phosphite, and
diphenyl(p-tolyl)phosphite.

6. A process according to claim 5, in which triphenylphosphite is used.

7. A process as claimed in any one of claims 1 to 6, in which the triphenylphosphite is used in an excess amount of from 3-5 moles per mole of rhodium compound.

8. A process as claimed in any one of the claims 1 to 7, in which the temperature is in the range of from 55 to 75°C.

9. A process as claimed in any one of the claims 1 to 8, in which the pressure is in the range of from 15 to 60 bars.

10. A process as claimed in any one of claims 1 to 9, in which an apolar solvent selected from toluene, benzene and xylenes is used.

## Patentansprüche

1. Verfahren zur Hydroformylierung einer Verbindung der allgemeinen Formel

$$R_1 - CH = \overset{\overset{H}{|}}{C} - \overset{\overset{O}{\|}}{C} - O - R_2 \qquad I$$

in der $R_1$ ein Wasserstoffatom oder eine Alkylgruppe mit 1 bis 6 Kohlenstoffatomen bedeutet und $R_2$ eine Alkylgruppe mit 1 bis 18 Kohlenstoffatomen bedeutet, umfassend die Hydroformylierung der Verbindung der allgemeinen Formel I mit Kohlenmonoxid und Wasserstoff in flüssiger Phase in Gegenwart eines homogenen Katalysatorsystems, umfassend

a) eine Rhodiumverbindung und

b) ein oder mehrere Triphenylphosphite, deren Phenylgruppen gegebenenfalls substituiert sein können durch Halogen, Alkyl mit 1 bis 6 Kohlenstoffatomen, Alkoxy mit 1 bis 6 Kohlenstoffatomen, Carboxy oder Cyano, bei einer Temperatur im Bereich von 50 bis 100°C und einem Druck im Bereich von 3 bis kleiner 100 bar.

2. Verfahren nach Anspruch 1, wobei $R_1$ ein Wasserstoffatom bedeutet.

3. Verfahren nach Anspruch 2, wobei $R_2$ eine Methylgruppe bedeutet.

4. Verfahren nach einem der Ansprüche 1 bis 3, wobei die Anzahl gAtom Rhodium, das verwendet wird, pro mol Verbindung der allgemeinen Formel I im Bereich von 0,001 bis 0,1 liegt.

5. Verfahren nach einem der Ansprüche 1 bis 4, wobei das oder jedes angewandte Triphenylphosphit ausgewählt ist aus : Triphenylphosphit, Diphenyl-(p-chlorphenyl)phosphit, Diphenyl-(p-methoxyphenyl)phosphit, Diphenyl-(p-cyanophenyl)phosphit und Diphenyl-(p-tolyl)phosphit.

6. Verfahren nach Anspruch 5, wobei Triphenylphosphit verwendet wird.

7. Verfahren nach einem der Ansprüche 1 bis 6, wobei das Triphenylphosphit in einem Überschuß von 3 bis 5 mol pro mol Rhodiumverbindung angewandt wird.

8. Verfahren nach einem der Ansprüche 1 bis 7, wobei die Temperatur im Bereich von 55 bis 75°C liegt.

9. Verfahren nach einem der Ansprüche 1 bis 8, wobei der Druck im Bereich von 15 bis 60 bar liegt.

10. Verfahren nach einem der Ansprüche 1 bis 9, wobei ein apolares Lösungsmittel, ausgewählt aus Toluol, Benzol und Xylolen, verwendet wird.

## Revendications

1. Un procédé pour l'hydroformylation d'un composé de formule générale

$$R_1 - CH = \overset{\overset{H}{|}}{C} - \overset{\overset{O}{\|}}{C} - O - R_2 \qquad I$$

dans laquelle $R_1$ représente un atome d'hydrogène et un groupe alkyle présentant de 1 à 6 atomes de carbone et $R_2$ représente un groupe alkyle présentant de 1 à 18 atomes de carbone, procédé selon lequel on réalise l'hydroformylation du composé de formule générale I avec du monoxyde de carbone et de l'hydrogène en phase liquide en présence d'un système catalytique homogène comportant :

a) un composé du rhodium et

b) un ou plus d'un triphénylphosphite, dont les groupes phényles peuvent être facultativement substitués par de l'halogène, un alkyle présentant de 1 à 6 atomes de carbone, un alcoxy présentant de 1 à 6 atomes de carbone, du carboxy ou du cyano, à une température dans la gamme de 50 à 100°C et sous une pression dans la gamme de 3 à moins de 100 bars.

2. Un procédé tel que revendiqué dans la revendication 1, dans lequel $R_1$ représente un atome d'hydrogène.

3. Un procédé tel que revendiqué dans la revendication 2, dans lequel $R_2$ représente un groupe méthyle.

4. Un procédé tel que revendiqué dans l'une quelconque des revendications 1 à 3, dans lequel le nombre d'atomes grammes de rhodium utilisé par mole de composé de formule générale I se situe dans la gamme de 0,001 à 0,1.

5. Un procédé tel que revendiqué dans l'une quelconque des revendications 1 à 4, dans lequel le ou chaque triphénylphosphite utilisé est choisi parmi :
le triphénylphosphite,
le diphényl(p-chlorophényl)phosphite
le diphényl(p-méthoxyphényl)phosphite,
le diphényl(p-cyanophényl)phosphite, et
le diphényl(p-tolyl)phosphite.

6. Un procédé selon la revendication 5, dans lequel le triphénylphosphite est utilisé.

7. Un procédé tel que revendiqué dans l'une quelconque des revendications 1 à 6, dans lequel le triphénylphosphite est utilisé en une quantité excédentaire de 3 à 5 moles par mole de composé du rhodium.

8. Un procédé tel que revendiqué dans l'une quelconque des revendications 1 à 7, dans lequel la température se situe dans la gamme de 55 à 75°C.

9. Un procédé tel que revendiqué dans l'une quelconque des revendications 1 à 8, dans lequel la pression se situe dans la gamme de 15 à 60 bars.

10. Un procédé tel que revendiqué dans l'une quelconque des revendications 1 à 9, dans lequel un solvant apolaire choisi parmi le toluène, le benzène et les xylènes est utilisé.